# EUROPEAN PATENT APPLICATION

(11) **EP 3 888 582 A1**
(43) Date of publication of application: **06.10.2021**
(21) Application number: 21166043.6
(22) Date of filing: 30.03.2021
(51) Int. Cl.: A61B 34/20, A61B 17/70, A61B 34/10, A61B 90/00, A61B 17/00

(54) **NON-OPTICAL NAVIGATION SYSTEM FOR GUIDING TRAJECTORIES**

(30) Priority: 30.03.2020 US 202063001841 P
(71) Applicant: Zimmer Biomet Spine, Inc., Westminster, Colorado 80021 (US)
(72) Inventor: SCHLOSSER, Jeffrey, Menlo Park, CA 94025 (US)
(74) Representative: Mays, Julie

(57) **Abstract**

A system or method may be used to align a guide (e.g., a screw guide, a trajectory guide for biopsies, etc.) for insertion of a screw, instrument, or tool (e.g., a spinal screw) at a specified angle. The system and method may track the movement and angle of the guide which may be affixed to an articulated arm with respect to a reference point based on sensor data. The system and method may determine whether the guide is at the specified angle based on sensor data.

## Description

### CLAIM OF PRIORITY

This application claims the benefit of priority to U.S. Provisional Application No. 63/001,841, filed March 30, 2020, titled "NON-OPTICAL NAVIGATION SYSTEM FOR GUIDING TRAJECTORIES."

### BACKGROUND

Some surgical procedures require particular insertion trajectories for tools or instruments, for example based on patient anatomy, the specific tool or instrument, and the procedure being done. Navigating and stabilizing surgical trajectories is typically done by hand and eye of the surgeon, or with mechanical guides. These techniques do not always produce reliable repeatability, or are dependent on skilled surgeons having years of experience.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a view of a navigation system including an articulated arm, in accordance with some embodiments.
FIGS. 2A-2E illustrate a tool insertion guide system in various configurations, in accordance with some embodiments.
FIG. 3 illustrates a sensor device including at least one marker, in accordance with some embodiments.
FIG. 4 illustrates a graphic user interface (GUI) displaying angle or position information corresponding to aspects of an articulated arm, in accordance with some embodiments.
FIG. 5 illustrates a detailed view of a screw guide including a set of LEDs, in accordance with some embodiments.
FIG. 6 illustrates a diagram showing a screw guide registration technique, in accordance with some embodiments.
FIGS. 7A-7C illustrate example depth sensor configurations, in accordance with some embodiments.
FIG. 8 illustrates a flowchart showing a technique for aligning a screw guide for inserting a spinal screw at a specified angle, in accordance with some embodiments.
FIG. 9 illustrates a block diagram of an example machine upon which any one or more of the techniques discussed herein may perform in accordance with some embodiments.

### DETAILED DESCRIPTION

Systems and methods for navigating and stabilizing surgical trajectories are described herein. The systems and methods may be used for aligning a screw guide for inserting a screw at a specified angle are described herein. For example, in a spinal screw insertion, an angle may be specified with respect to patient anatomy (e.g., a spine bone or vertebrae) and a screw guide may be used to insert the screw at the specified angle. In techniques that do not use a screw guide or use a handheld screw guide, locating and maintaining the specified angle for any period of time may be difficult. In the presently described systems and methods procedures, an adjustable articulated arm may be used to hold the screw guide at the specified angle. Although the examples herein are described with respect to spinal screw insertion, the systems and methods may be used for biopsy trajectories, trajectories for ablation probes (e.g., RF, Microwave, Cryo), or the like.

The articulated arm may be operated with a controller (e.g., processing circuitry), which may be used to lock the articulated arm in a particular position or orientation. In an example, the controller may lock the articulated arm in response to detecting that the screw guide is aligned with the specified angle. In another example, a user (e.g., a surgeon) may lock the articulated arm manually. A user interface (UI), such as a display screen or set of (light emitting diodes) LEDs may be used to communicate a current angle of the articulated arm to the user or provide other feedback.

In an example, the articulated arm may be manually articulable when unlocked and prevented from moving when locked. The systems and methods described herein may be used for tracking an articulated arm, determining an angle or orientation of the articulated arm (or a component attached to the articulated arm), and locking the articulated arm at a specified angle.

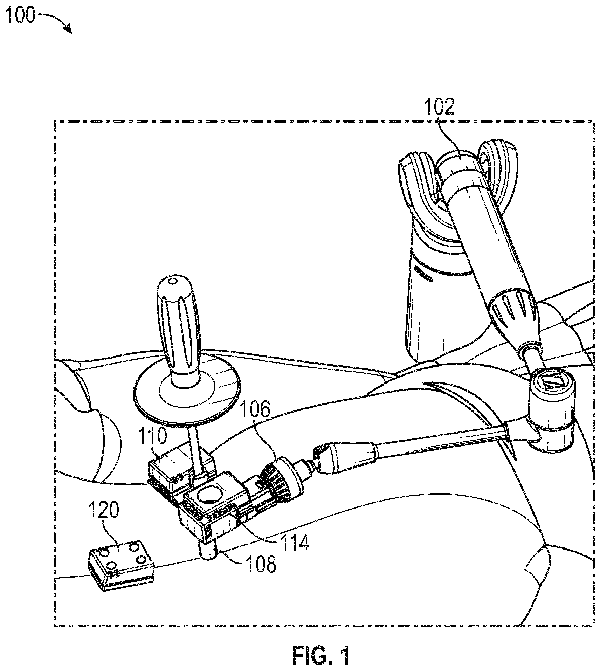

FIG. 1 illustrates a view of a navigation system 100 including an articulated arm 102, in accordance with some embodiments. The articulated arm 102 may include two or more segments, connected by at least one joint. In an example, the segments may be independently moveable. The articulated arm 102 includes an end effector 106 affixed to a most distal segment (optionally rigidly or via a joint). The end effector 106 includes a screw guide 108 (e.g., the screw guide may be the end effector, the screw guide may be affixed or coupled to a portion of an end effector, or the like). The screw guide 108 may include a sensor 110, described in further detail below.

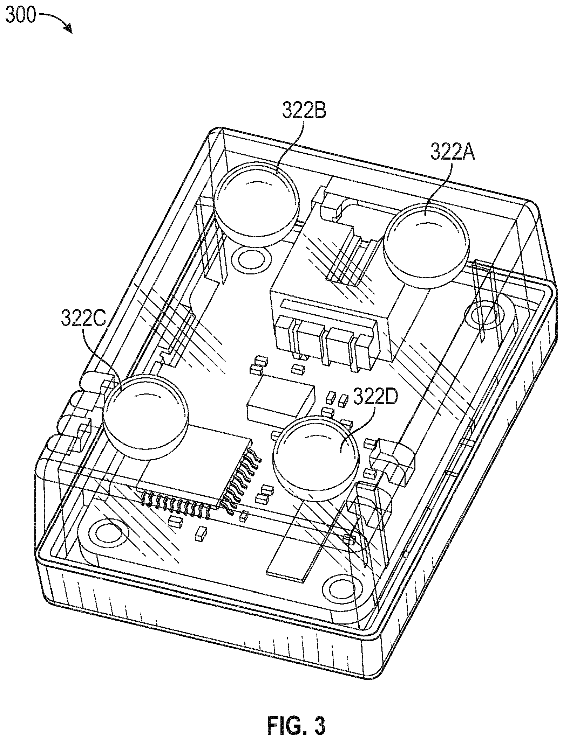

In some examples, the sensor 110 may include one or more of an accelerometer, a gyroscope, or a magnetometer (e.g., an inertial measurement unit (IMU)). The sensor 110 may be used to track the orientation or position of the screw guide 108 as the articulated arm 102 is moved. The orientation or position of the screw guide 108 may be tracked relative to a base portion of the articulated arm 102, relative to an absolute position or orientation (e.g., gravity for orientation, a fixed location in a room for position, etc.), or relative to a patient (e.g., via a fixed bed). The relative location may be determined by comparing sensor data from the sensor 110 to data from a second sensor (e.g., sensor 120), which may be affixed to the patient, a bed holding the patient, a base of the articulated arm 102, a location within a room, or the like. The second sensor 120 may be the same type of sensor as the first sensor 110 or they may differ (e.g., both may be IMUs, or one may be an IMU with the other being an accelerometer, or the like). A registration technique may be used to register the sensor 110 or the sensor 120 to an image of the patient, in an example. The registration may be used to register an output on a user interface in an example. The sensor 120 may be secured in some examples by a spinous process clamp, a sacral screw fixation, a non-invasive strap or glue, or the like. The sensor 120 may include one or more markers (e.g., a radiopaque marker) for registration, such as is described below with respect to FIG. 3.

In some examples, a depth sensor may be affixed to the screw guide 108. In some examples, the depth sensor may be a position encoder, an optical flow sensor, a magnetic sensing system, a hall effect magnetic sensor, a linear optical encoder, an infrared sensor, a laser, a mechanical mechanism, or the like. The depth sensor may be used to determine an insertion depth of an instrument (e.g., a tool, a drill, a testing apparatus, a screw, etc.).

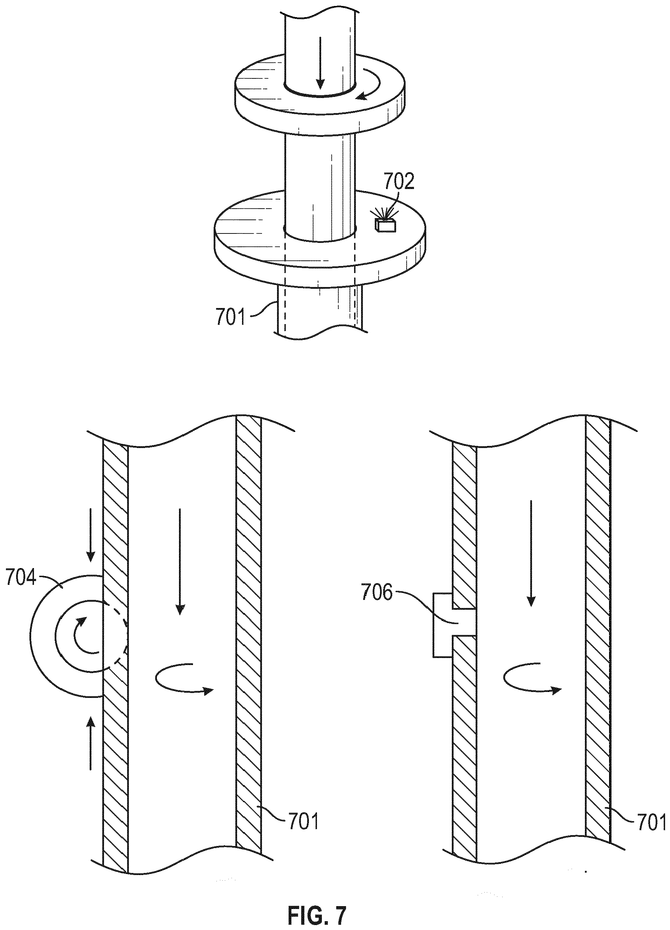

In some examples, an LED or a plurality of LEDs 114 (e.g., as shown in FIG. 1) may be affixed to screw guide 108. In some examples, one or more of the plurality of LEDs 114 may be used to indicate a current position or orientation of the screw guide 108 or to indicate a direction of movement or angle needed to place the screw guide 108 at a specified angle or in a specified position. The LEDs are described in further detail below with respect to FIG. 7. In another example, a graphical user interface may be used to indicate a current position or orientation or indicate directions to achieve a specified orientation or position. In other examples, haptic, audible, or other visual feedback may be used to provide position or orientation information or to provide feedback about directions to move the screw guide 108 to achieve a specified orientation or position.

The navigation system 100 may include processing circuitry to determine whether the screw guide 108 is configured at a specified orientation or position. The processing circuitry may further cause a locking mechanism to lock the articulated arm 102 (e.g., and the screw guide 108) in place when the screw guide 108 is at the specified orientation or position. The locking mechanism or mechanisms may be powered and may optionally resist movement. In some examples, the locking mechanism may include electro-mechanical locks within one or more joints of the articulated arm 102. In further examples, the articulated arm 102 may include one or more actuators, servos, or other motors operable to move one or more portions of the arm in response to a control signal. The processing circuitry may operate a controller to cause the articulated arm 102 to move or lock, in an example. The processing circuitry may be incorporated into the articulated arm 102 (e.g., at a base of the articulated arm 102) or may be remote from the articulated arm 102 (e.g., a computer, a mobile device, a server, etc.).

The navigation system 100 may be used for preparing a surgical procedure. For example, a screw entry point, a trajectory, or a depth for a screw placement (e.g., in the spine) may be preplanned using a diagnostic image (e.g., a CT or MRI) of a patient. The sensors 110 or 120 may be calibrated, with the sensor 120 secured to the patient, for example, and the sensor 110 secured to the end effector 106. An interoperative diagnostic image (e.g., CT or x-ray) of the patient may be obtained for use in registering the sensor 120 to the preoperative diagnostic image captured. The sensor 120 may include markers for reference in the two diagnostic images. In an example, the preparation may include selecting a first preplanned screw trajectory (e.g., on a user interface), accessing a pedicle, and identifying a screw entry point. The screw guide 108 may be placed against pedicle bone at a specified entry point (e.g., using the user interface for reference, using the articulated arm 102 as a robotic arm, or the like). The screw guide 108 may be moved by moving the articulated arm 102, for example according to feedback given on a user interface, via the LEDs 114, or via other feedback, until the screw guide 108 is aligned with a preplanned axial and sagittal angle. The articulated arm 102 may be automatically locked such that the screw guide 108 does not move (e.g., the articulated arm 102 is locked against movement by gravity, or optionally another force) when the screw guide 108 is aligned with the preplanned angles. After preparing the surgical procedure, the surgical procedure may be performed, for example by drilling, tapping, and placing screws. An instrument may be used to ensure holes are drilled to correct depths and screws are placed at appropriated depths.

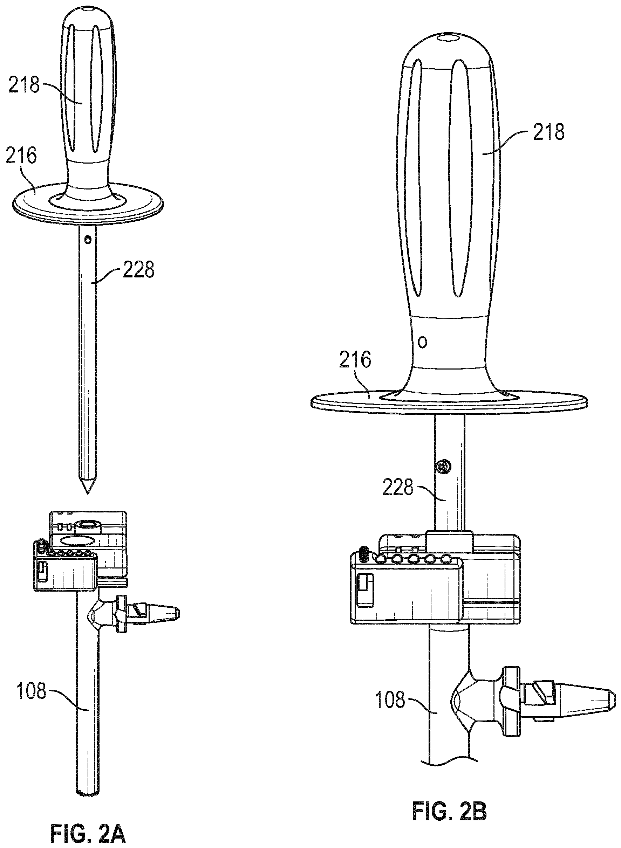

FIGS. 2A-2E illustrate a tool insertion guide system in various configurations, in accordance with some embodiments. FIGS. 2A and 2B show an instrument 218 before and after initial insertion into a screw guide (e.g., screw guide 108 of FIG. 1), respectively. The instrument 218 may have a lip 216 that may be used with a depth sensor to track the depth to which a shaft 228 of the instrument 218 has been inserted into the screw guide 108. The lip 216 may be a flange, guard, depth-limiter, or the like, and the lip 216 may be used to prevent over-insertion of the instrument 218 into the screw guide 108 or into a patient. The instrument 218 may be a screw driver, a drill, a pointer, or the like.

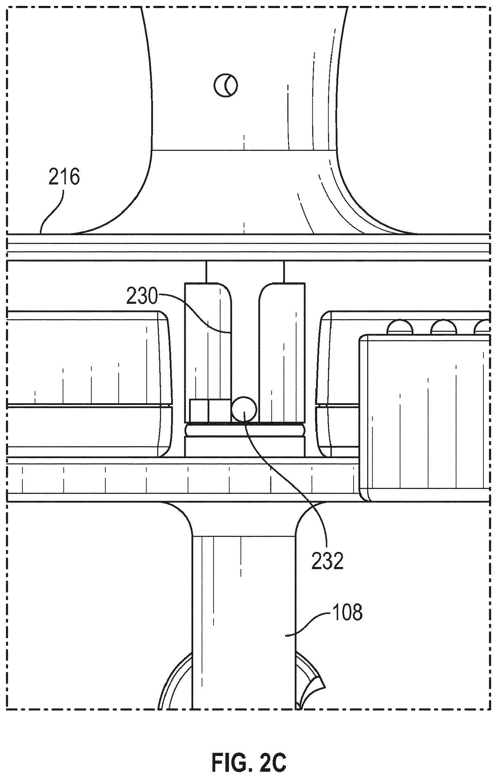

In an example, the instrument 218 may be a bone pointer, which may be slid into the screw guide. Once inserted, the instrument 218 may be locked into the screw guide 108, as shown in FIG. 2C. For example, the instrument 218 may be rotated such that a pin 232 of the instrument 218 may be locked into an L-shaped slot 230 of the screw guide 108. The L-shaped slot 230 may include a vertical portion and a horizontal portion, in which the pin 232 may be aligned for full insertion along the vertical portion of L-shaped slot 230, and locked via the horizontal portion. In this example, when the pin 232 is fully inserted into the vertical portion of the L-shaped slot 230, the instrument 218 may be rotated such that the pin 228 travels along the horizontal portion of the L-shaped slot 230, locking the instrument 218 in place (e.g., helping to limit or resist vertical movement of the instrument 218).

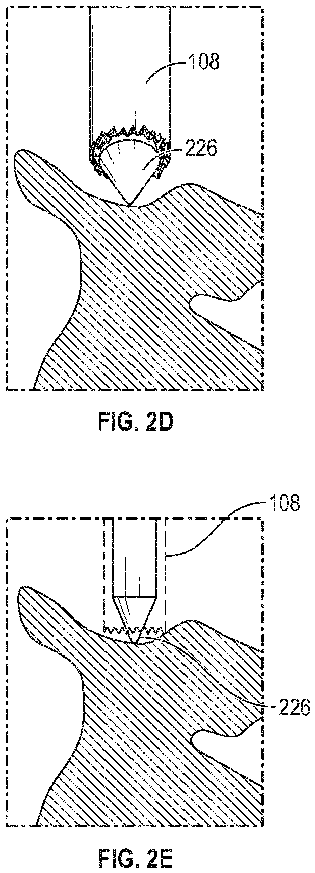

As shown in FIGS. 2D-2E, a tip 226 of the instrument 218 may extend out of the shaft of the screw guide. The tip 226 may be placed against the bone at a pedicle screw entry point, for example after instrument 218 is locked in the L-shaped slot. When the tip 226 makes contact with bone, the instrument 218 may be unlocked (e.g., by being rotated in an opposite direction of the previous horizontal locking direction), and slid along the screw guide. The surface of the screw guide that makes contact with the bone may be rough or knurled. This surface may be used to prevent movement of the screw guide with respect to the pedicle surface, which prevents skiving of the screw, a common concern during spine surgery.

In an example, the screw guide 108 may automatically move to contact the pedicle, as shown in FIG. 2E, as the instrument 218 is removed. In another example, the screw guide may be moved down until it contacts the pedicle when the instrument 218 is removed. Thus, the instrument 218 may be used to more precisely identify an entry point (e.g., using the tip 226) versus only using the screw guide and attempting to center the shaft of the screw guide over the entry point. After the screw guide contacts bone around the entry point, the instrument 218 may be fully removed from the screw guide such that a drill or screwdriver may be inserted (e.g., after a trajectory of the screw guide is locked, such as an orientation corresponding to a specified angle, as described herein). In some examples, instrument 218 may be a bone pointer, a drill, a screwdriver, or the like.

In other examples, a spring may be used between the instrument 218 and the screw guide. In this example, the instrument 218 pushes on the spring when locating the screw entry point, and the instrument 218 is released when the point is found, allowing the spring to expand and the screw guide to touch bone. The location of the pedicle screw entry point may be made manually or automatically. In the automatic example, the entry point may be determined using a preoperative diagnostic image (e.g., CT or MRI) along with a machine learning technique (e.g., trained using a database of pedicle screw placements). In an example, a camera-based system may be used to identify the preplanned entry point based on local bone topography, for example with a laser or other indicator.

FIG. 3 illustrates a sensor device 300 including at least one marker, in accordance with some embodiments. In an example, the sensor device 300 may be the sensor 110 or the sensor 120 of FIG. 1. The sensor device 300 may, in some examples, include an accelerometer, a gyroscope, or magnetometer (e.g., an IMU). The sensor device 300 may be secured to a patient, a bed, a base of an articulated arm, an end effector, etc. by a spinous process clamp, a sacral screw fixation, a non-invasive strap or glue, or the like.

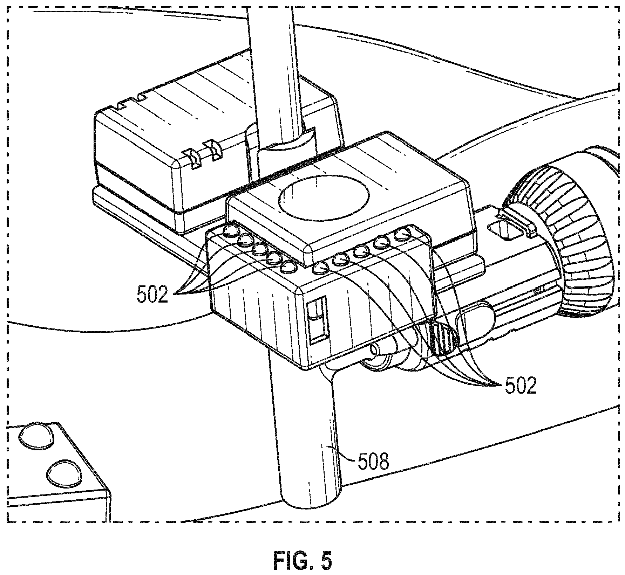

The sensor device 300 includes at least one marker (e.g., radio-opaque markers 322A-322D). The cover or housing of the sensor device 300 may be radiolucent such that the radio-opaque markers may be identified on a diagnostic image. The markers may be affixed to a housing of the sensor device 300 or housed within the sensor device 300. A preoperative diagnostic image may be taken of patient anatomy, including the sensor device 300 placed or attached to a stationary object (e.g., a bed the patient is on). This preoperative diagnostic image may be an x-ray, a CT, an MRI, or the like. The preoperative diagnostic image may be captured such that a marker (e.g., 322A-322D) is visible on the preoperative diagnostic image. The marker may be used to register an intraoperative patient diagnostic image. The marker placement may be used to determine a relative location of a second sensor device (e.g., affixed to an end effector of an articulated arm, such as sensor 110 of FIG. 1). Further registration details are described below with respect to FIG. 5. FIG. 3 illustrates four radio-opaque markers, 322A-D, the plurality of which may be useful for defining a coordinate system, although more or fewer markers may be used with the techniques and systems described herein.

The markers (e.g., 322A-322D) may be visible on a CT or x-ray image, and stationary with respect to the patient when the housing of the sensor device 300 is affixed or placed on a fixed surface (e.g., a patient bed). The sensor device 300 may include a 9 degree of freedom sensor chip with an accelerometer, a gyroscope, and a magnetometer, in an example.

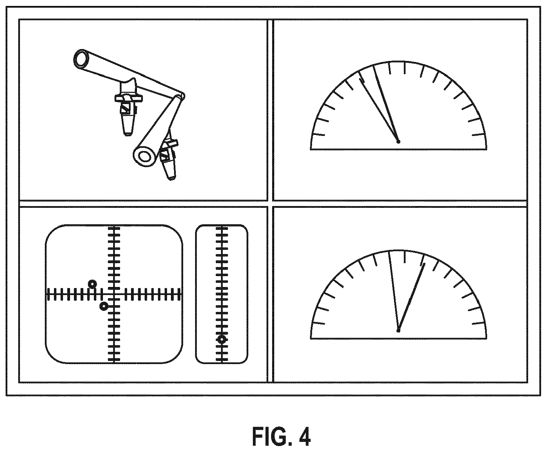

FIG. 4 illustrates a graphic user interface (GUI) 400 displaying angle or position information corresponding to aspects of an articulated arm, in accordance with some embodiments. The GUI 400 includes one or more representations of a specified orientation (e.g., a preplanned trajectory for a screw entry) and a current orientation of a screw guide (e.g., affixed to the articulated arm). The GUI 400 shown in FIG. 4 illustrates a few example user interface components, although fewer may be used, or different design choices may be made without deviating from the systems and methods described herein. For example, GUI 400 includes four different quadrants including an upper left quadrant showing a simplified diagram of a virtual screw guide at a preplanned angle and a current orientation of a physical screw guide. The right two quadrants of GUI 400 show an axial angle and a lateral angle, for example with respect to a vertical angle (e.g., directly opposite gravity). Other orientations of these angles (e.g., with respect to a horizon or arbitrary angle) may be used. The bottom left quadrant shows a reticle (e.g., crosshairs) with a preplanned orientation and a current orientation. Any of these three examples may be sufficient for guiding a user to placing the screw guide at the specified orientation. As the screw guide is moved (e.g., by moving an articulated arm that the screw guide is affixed to), the current orientation may move on the one or more quadrants. When the screw guide is aligned with the specified orientation, an alert may be displayed on the GUI 400 (e.g., indicating the articulated arm is now locked or asking whether the articulated arm is to be locked in place). In the example GUI 400 of FIG. 4., the bottom left quadrant includes a singular axis that may be used to represent depth of insertion of an instrument, tool, or screw.

In some examples, the GUI 400 may display a real-time relative angle between the screw guide (e.g., screw guide 108 of FIG. 1) with respect to a specified angle (e.g., a preplanned pedicle screw insertion angle). In some examples, the GUI 400 displays data of the real-time relative angle of the screw guide as determined by processing circuitry within a registered coordinate system. In some examples, GUI 400 may display a three-dimensional visual representation of the screw guide body and the real-time relative angle of the screw guide. In some examples, GUI 400 may display the real-time relative angle of the screw guide on a coordinate grid. In some examples, GUI 400 may display one or more gauges displaying the relative angle of the screw guide. The GUI 400 may indicate the current axial angle, sagittal angle, and depth relative to the preplanned values.

FIG. 5 illustrates a detailed view of a screw guide 508 (e.g., screw guide 108 of FIG. 1) including a set of LEDs 502 (e.g., LEDs 114 of FIG. 1), in accordance with some embodiments. Although a plurality of LEDs 502 are shown in FIG. 5, as few as a single LED may be used to implement the techniques described herein. One or more LEDs 502 may be affixed or attached to screw guide 508. In some examples, the one or more LEDs 502 may indicate a current angular orientation or an indication of how far or in which direction to move screw guide 508 to achieve a specified angle (e.g., a preplanned screw entry angle). In some examples, the one or more LEDs 502 may be arranged in two perpendicular (or non-parallel) rows, as illustrated in FIG. 5. In this and similar examples, each row may represent a respective lateral or axial direction.

In an example not shown in FIG. 5, the one or more LEDs 502 may be arranged in a single row. In a first configuration of this example, the single row of one or more LEDs 502 may be centered around a middle LED with at least one LED on each side representing respective lateral or axial directions. In another example, the center LED may turn a particular color when the specified angle is achieved, or the center LED may turn on or off when the specified angle is achieved. In a second configuration of the single row example, there may be an even number of the one or more LEDs, with each half of the single row representing respective lateral or axial directions. In some examples of this second configuration, the one or more LEDs 502 may turn off completely when the specified angle is achieved, or all of the one or more LEDS 502 may turn on when the specified angle is achieved.

In an example, the one or more LEDs 502 may dim or brighten to indicate which direction or how far to move the screw guide 508 to achieve the specified angle. In a first configuration of this example, each of the one or more LEDs 502 may have three levels of brightness (e.g., bright, medium, and dim) as well as completely off. In another example, the one or more LEDs 502 may have two brightness levels (e.g., bright and dim) as well as completely off. In other examples, a spectrum of brightness may be used. In an example of this first configuration, bright may mean close, medium may mean closer, or dim may mean even closer, with the adjacent LED(s) (if any) continuing the process until the specified angle is achieved. In this example, the last LED being off or being on may signal the specified lateral or axial angle has been achieved. In a second configuration of the brightness level example, the one or more LEDs 502 may include two LEDs, each representing respective axial or lateral directions, and each having a steady spectrum of brightness culminating in each LED turning completely off when the specified angle is achieved. In other examples of the first and second configuration, brightness may be the indicator of proximity or achievement of the specified angle. In some examples of the first or second configuration, a second type of feedback may accompany the one or more LEDs 502 turning completely off or fully on, such as haptic, audio, or other feedback.

In some examples, one or more of the set of LEDs 502 may have different colors or may all have the same color. In an example, the one or more LEDs 502 may change color when the screw guide 508 is moved closer to or farther away from the specified orientation. In a well-lit surgical theater, it may be beneficial to have a secondary indication that the specified angle has been achieved or is closer to being achieved in addition to a light-based indication. In some examples, a secondary indication may be audible, haptic, or visual.

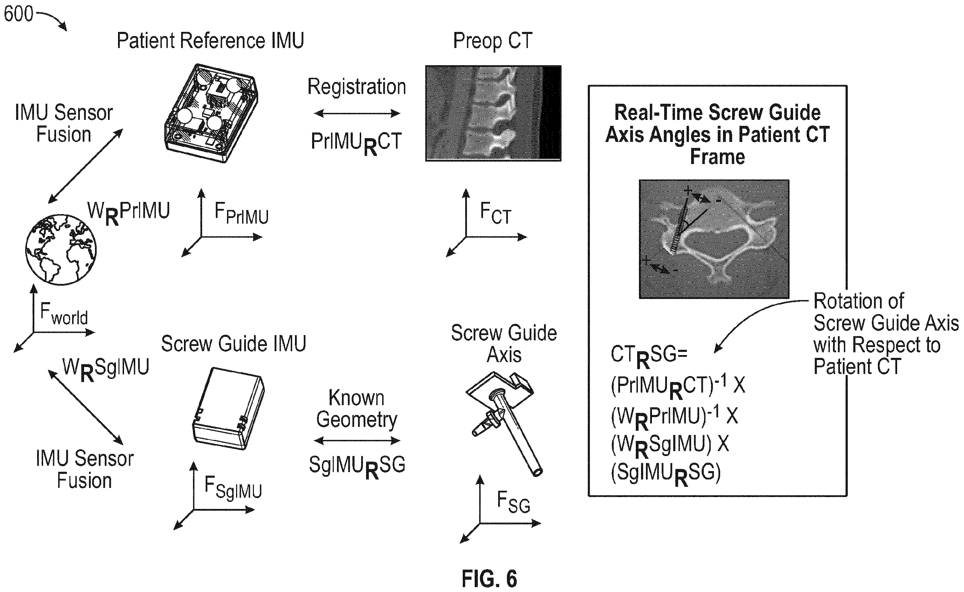

FIG. 6 illustrates a diagram 600 showing a screw guide registration technique, in accordance with some embodiments. The screw guide registration technique may be used to register a frame of reference of a preoperative diagnostic image to an intraoperative frame of reference such that a current orientation of a screw guide may be tracked with respect to patient anatomy. The preoperative diagnostic image frame of reference F_{CT} may be registered using a patient reference sensor (e.g., an IMU, such as sensor 300 of FIG. 3) that is fixed to a patient anatomy or to a base of an articulated arm (e.g., via a rigid structure, such as a bed the patient is on). The patient reference sensor has a frame of reference F_{PRIMU} that may be registered to the preoperative diagnostic image based on one or more markers (e.g., radio-opaque markers) within or affixed to the housing of the patient reference sensor.

Within an intraoperative frame of reference, a screw guide reference frame F_{SG} (e.g., related to the screw guide 108 of FIG. 1) may be translated to a screw guide sensor (e.g., an IMU) frame F_{SGIMU} based on known geometry connecting the screw guide sensor to the screw guide (and a known axis of the screw guide). Based on sensor data received from the patient reference sensor and the screw guide sensor, a sensor fusion may be performed to connect the frames of reference (e.g., the screw guide axis may be registered to the preoperative diagnostic image). The sensor fusion relates the patient reference sensor to the screw guide sensor. The frame of reference connection via the sensor fusion enables precise sensing of axial and sagittal screw angles of the screw guide axis with respect to the angles planned on the preoperative diagnostic image.

In an example, known geometry connecting the screw guide or the screw guide sensor to the patient reference IMU may be used instead of or in addition to the sensor fusion. The known geometry may be based on geometry of an articulated arm, in an example where the articulated arm may output angular information of its segments.

FIGS. 7A-C illustrate example depth sensor configurations, in accordance with some embodiments. FIG. 7A illustrates a laser sensor 702 affixed to a top surface of a screw guide 701 that is orthogonal to an insertion shaft of the screw guide 701. When an instrument, tool, or screw is inserted into the insertion shaft, the laser sensor 702 may output sensor information indicating a distance from the laser sensor 702 to a portion of the instrument, tool, or screw, such as a lip (e.g., lip 216). In some examples, sensor 702 is attached to a top horizontal surface of screw guide 701. Laser sensor 702 may measure the distance that an instrument has been inserted into screw guide 701 by detecting the movement of the lip of the instrument.

FIG. 7B illustrates a rotary encoder sensor 704 that is affixed to a shaft wall of the screw guide 701. The wheel of the rotary encoder sensor 704 may be rubber, or other material. As an instrument, tool, or screw is inserted into the insertion shaft, the rotary encoder sensor 704 may rotate. During or after rotation, the rotary encoder sensor 704 may output sensor information indicating a distance that the instrument, tool, or screw has traversed past the rotary encoder sensor 704, corresponding to a distance that the rotary encoder sensor 704 has rotated. The distance traversed may be used to determine an insertion depth of the instrument, tool, or screw.

FIG. 7C illustrates an optical flow sensor 706 embedded in a shaft wall of the screw guide 701. The optical flow sensor 706 tracks movement detected along its face. For example, when an instrument, tool, or screw is inserted into the insertion shaft, the optical flow sensor 706 may track the movement. The optical flow sensor 706 outputs sensor information indicating a distance that the instrument, tool, or screw has traversed past the optical flow sensor 706. The distance traversed may be used to determine an insertion depth of the instrument, tool, or screw.

In other examples, a depth sensor may include a position encoder, a magnetic sensing system, a hall effect magnetic sensor, a linear optical encoder, an infrared sensor, a mechanical mechanism, or the like. For example, a magnetic sensing system with multiple interleaving magnets or sensors may be used, similar to how a digital caliper works. In this example, a magnetic strip on each instrument to be tracked may be applied. In another example, a Hall effect magnetic sensor may be used, for example with a magnet on each instrument to be tracked.

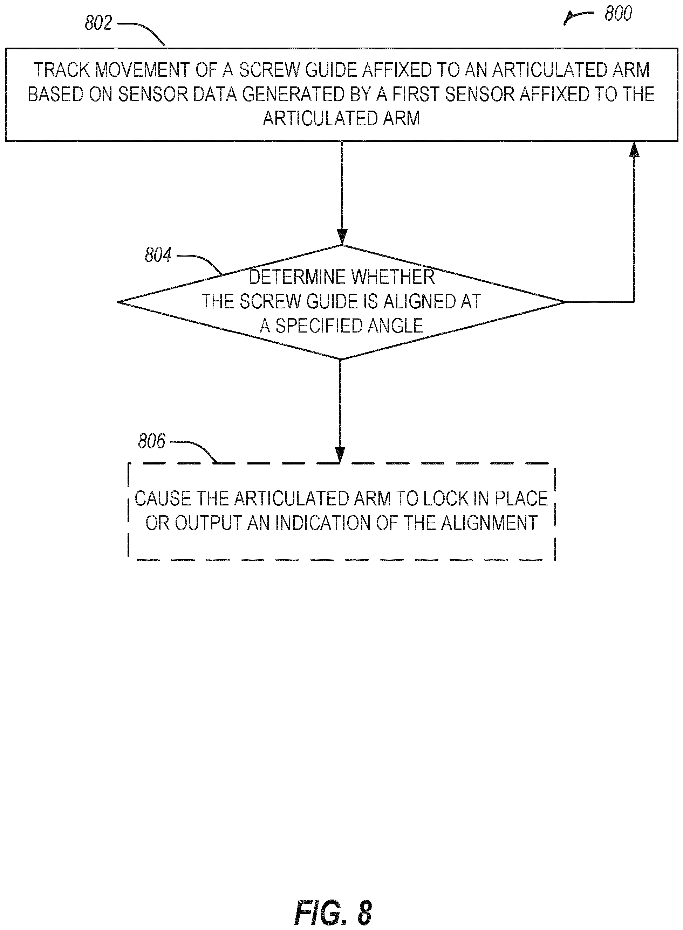

FIG. 8 illustrates a flowchart illustrating a technique 800 for aligning a screw guide for inserting a spinal screw at a specified angle, in accordance with some embodiments. The technique 800 may be performed by processing circuitry, for example by implementing a controller of an articulated arm, a locking mechanism, or other components, devices, or systems described in the technique 800. The technique 800 as discussed includes operations that may be performed by multiple different actors, devices, or systems.

The technique 800 includes an operation 802 to track movement of a screw guide affixed to an articulated arm based on sensor data generated by a first sensor affixed to the articulated arm. The screw guide may be fixed to a distal end of the articulated arm. In an example, the movement of the screw guide is tracked with respect to a reference point (e.g., a point identified by a second sensor). Tracking may include receiving information indicative of the reference point from a second sensor secured to a patient or a fixed object, such as a bed. In an example, tracking may include receiving accelerometer data, gyroscope data, or magnetometer data from the first sensor, where for example, the first sensor is an IMU.

The technique 800 includes a decision operation 804 to determine the screw guide is aligned at a specified angle (e.g., whether the articulated arm is at an orientation such that the screw guide is aligned at the specified angle). The specified angle may be a preplanned angle for inserting a screw at an entry point (e.g., a patient spine). In response to determining that the screw guide is not aligned at the specified angle, the technique 800 may return to operation 802 to continue tracking movement of the screw guide. In response to determining that the screw guide is aligned at the specified angle, the technique 800 may continue to operation 806.

The technique 800 includes an operation 806 to cause the articulated arm to lock in place or output an indication of the alignment. The indication of the alignment may be an alert, requesting whether a user confirms locking the articulated arm. The lock may occur automatically or with a confirmation.

The technique 800 may include registering the reference point to a preoperative patient image, for example based on a radio-opaque marker within a housing of the second sensor. The specified angle may be a screw entry angle preplanned using the preoperative patient image. The technique 800 may include receiving depth information, such as from a third sensor affixed to the screw guide, for a tool inserted into the screw guide. The depth information may correspond to a screw bore depth. The technique 800 may include displaying, on a GUI, a current angle of the screw guide and the specified angle of the screw guide during tracking. In an example, the technique 800 includes activating one or more of a plurality of LEDs affixed to the screw guide to indicate a relative direction from a current angle of the screw guide to the specified angle during tracking.

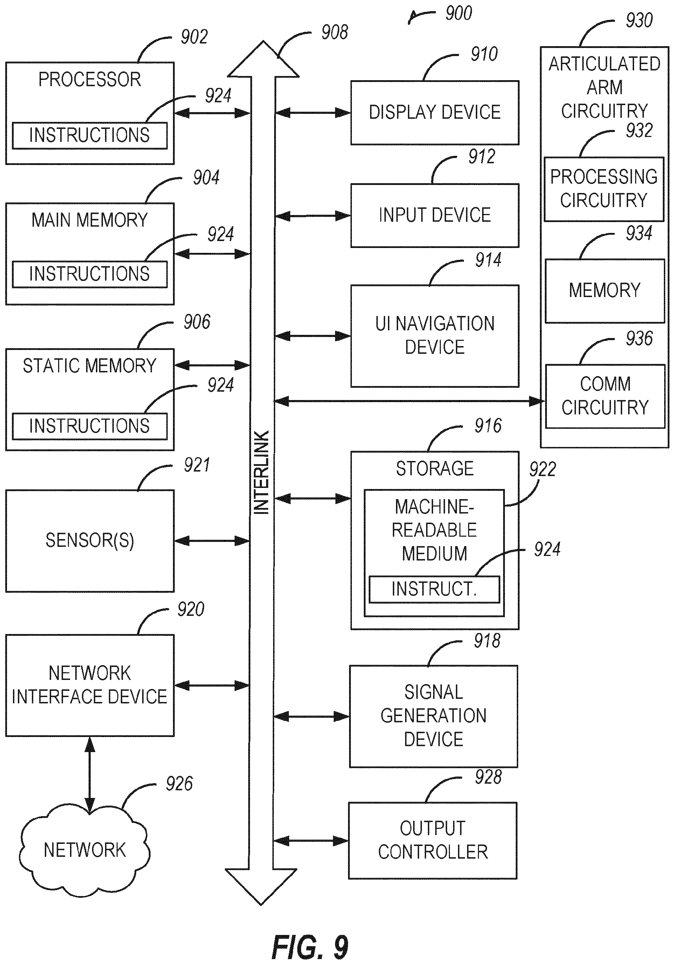

FIG. 9 illustrates a block diagram of an example machine 900 upon which any one or more of the techniques discussed herein may perform in accordance with some embodiments. In alternative embodiments, the machine 900 may operate as a standalone device or may be connected (e.g., networked) to other machines. In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 900 may include a hardware processor 902 (e.g., a central processing unit (CPU), a graphics processing unit (GPU), a hardware processor core, or any combination thereof), a main memory 904 and a static memory 906, some or all of which may communicate with each other via an interlink (e.g., bus) 908. The machine 900 may further include a display unit 910, an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensor. The machine 900 may include an output controller 928, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine readable medium 922 is illustrated as a single medium, the term "machine readable medium" may include a single medium or multiple media (e.g., a centralized or distributed database, and/or associated caches and servers) configured to store the one or more instructions 924. The term "machine readable medium" may include any medium that is capable of storing, encoding, or carrying instructions for execution by the machine 900 and that cause the machine 900 to perform any one or more of the techniques of the present disclosure, or that is capable of storing, encoding or carrying data structures used by or associated with such instructions. Non-limiting machine-readable medium examples may include solid-state memories, and optical and magnetic media.

The instructions 924 may further be transmitted or received over a communications network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi®, IEEE 802.16 family of standards known as WiMax®), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communications network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 900, and includes digital or analog communications signals or other intangible medium to facilitate communication of such software.

Machine 900 is connected to circuitry of an articulated arm 930. The articulated arm circuitry 930 may be connected to the machine 900 via a wired or wireless connection. Although shown connected to the interlink 908, the connection may be via the network 926 through the network interface device 920, the output controller 928, or other interface of the machine. The articulated arm circuitry 930 includes communication circuitry 936 for connecting to the machine 900. In an example, the communication circuitry 936 may include a wire, a wireless antenna and circuitry for wireless communications, interface circuitry, or the like. The articulated arm circuitry 930 may include processing circuitry 932 (e.g., one or more processors, a system on a chip, other hardwired circuitry, or the like) or memory 934. The articulated arm circuitry 930 may be housed in a base portion of an articulated arm (e.g., articulated arm 102 of FIG. 1). The processing circuitry 932 may be used to lock the articulated arm, unlock the articulated arm, identify that a current orientation of a screw guide (e.g., screw guide 108 of FIG. 1) corresponds to a specified orientation, or the like. The processing circuitry 932 may be used to perform the operations of technique 800 of FIG. 8, according to an example.

Each of these non-limiting examples may stand on its own, or may be combined in various permutations or combinations with one or more of the other examples.
Example 1 is a method for aligning a screw guide for inserting a spinal screw at a specified angle comprising: tracking, using processing circuitry, movement of the screw guide affixed to a distal end of an articulated arm with respect to a reference point based on sensor data generated by a first sensor affixed to the distal end of the articulated arm; determining, using the processing circuitry, whether the articulated arm is at an orientation such that the screw guide is arranged at the specified angle, based on the sensor data; and causing, in response to determining that the screw guide is arranged at the specified angle, a locking mechanism of the articulated arm to lock the articulated arm at the orientation.
In Example 2, the subject matter of Example 1 includes, wherein tracking the movement of the screw guide includes receiving information indicative of the reference point from a second sensor secured to a patient.
In Example 3, the subject matter of Example 2 includes, registering the reference point to a preoperative patient image based on a radio-opaque marker within a housing of the second sensor.
In Example 4, the subject matter of Example 3 includes, wherein the specified angle is a screw entry angle preplanned using the preoperative patient image.
In Example 5, the subject matter of Examples 1-4 includes, wherein tracking the movement includes receiving accelerometer data, gyroscope data, and magnetometer data from the first sensor, the first sensor being an inertial measurement unit (IMU).
In Example 6, the subject matter of Examples 1-5 includes, receiving depth information, from a third sensor affixed to the screw guide, for a tool inserted into the screw guide, the depth information corresponding to a screw bore depth.
In Example 7, the subject matter of Examples 1-6 includes, displaying, on a graphical user interface (GUI), a current angle of the screw guide and the specified angle of the screw guide during tracking.
In Example 8, the subject matter of Examples 1-7 includes, activating one or more of a plurality of light emitting diodes (LEDs) affixed to the screw guide to indicate a relative direction from a current angle of the screw guide to the specified angle during tracking.
Example 9 is a system for aligning a screw guide for inserting a spinal screw at a specified angle comprising: processing circuitry and memory, including instructions, which when executed by the processing circuitry, cause the processing circuitry to: track movement of the screw guide affixed to a distal end of an articulated arm with respect to a reference point based on sensor data generated by a first sensor affixed to the distal end of the articulated arm; determine whether the articulated arm is at an orientation such that the screw guide is arranged at the specified angle, based on the sensor data; and cause, in response to determining that the screw guide is arranged at the specified angle, a locking mechanism of the articulated arm to lock the articulated arm at the orientation.
In Example 10, the subject matter of Example 9 includes, wherein to track the movement of the screw guide, the processing circuitry is further configured to receive information indicative of the reference point from a second sensor secured to a patient.
In Example 11, the subject matter of Example 10 includes, wherein the instructions further cause the processing circuitry to register the reference point to a preoperative patient image based on a radio-opaque marker within a housing of the second sensor.
In Example 12, the subject matter of Example 11 includes, wherein the specified angle is a screw entry angle preplanned using the preoperative patient image.
In Example 13, the subject matter of Examples 9-12 includes, wherein to track the movement of the screw guide, the processing circuitry is further configured to receive accelerometer data, gyroscope data, and magnetometer data from the first sensor, the first sensor being an inertial measurement unit (IMU).
In Example 14, the subject matter of Examples 9-13 includes, wherein the instructions further cause the processing circuitry to receive depth information, from a third sensor affixed to the screw guide, for a tool inserted into the screw guide, the depth information corresponding to a screw bore depth.
In Example 15, the subject matter of Examples 9-14 includes, wherein the instructions further cause the processing circuitry to output for display, on a graphical user interface (GUI), an current angle of the screw guide and the specified angle of the screw guide during tracking.
In Example 16, the subject matter of Examples 9-15 includes, wherein the instructions further cause the processing circuitry to activate one or more of a plurality of light emitting diodes (LEDs) affixed to the screw guide to indicate a relative direction from a current angle of the screw guide to the specified angle during tracking.
Example 17 is at least one machine-readable medium, including instructions for aligning a screw guide for inserting a spinal screw at a specified angle, which when executed by processing circuitry, cause the processing circuitry to perform operations to: track movement of the screw guide affixed to a distal end of an articulated arm with respect to a reference point based on sensor data generated by a first sensor affixed to the distal end of the articulated arm; determine whether the articulated arm is at an orientation such that the screw guide is arranged at the specified angle, based on the sensor data; and cause, in response to determining that the screw guide is arranged at the specified angle, a locking mechanism of the articulated arm to lock the articulated arm at the orientation.
In Example 18, the subject matter of Example 17 includes, wherein to track the movement of the screw guide, the processing circuitry is further caused to receive information indicative of the reference point from a second sensor secured to a patient.
In Example 19, the subject matter of Example 18 includes, wherein the instructions further cause the processing circuitry to register the reference point to a preoperative patient image based on a radio-opaque marker within a housing of the second sensor.
In Example 20, the subject matter of Example 19 includes, wherein the specified angle is a screw entry angle preplanned using the preoperative patient image.
In Example 21, the subject matter of Examples 17-20 includes, wherein to track the movement of the screw guide, the processing circuitry is further configured to receive accelerometer data, gyroscope data, and magnetometer data from the first sensor, the first sensor being an inertial measurement unit (IMU).
In Example 22, the subject matter of Examples 17-21 includes, wherein the instructions further cause the processing circuitry to receive depth information, from a third sensor affixed to the screw guide, for a tool inserted into the screw guide, the depth information corresponding to a screw bore depth.
In Example 23, the subject matter of Examples 17-22 includes, wherein the instructions further cause the processing circuitry to output for display, on a graphical user interface (GUI), a current angle of the screw guide and the specified angle of the screw guide during tracking.
In Example 24, the subject matter of Examples 17-23 includes, wherein the instructions further cause the processing circuitry to activate one or more of a plurality of light emitting diodes (LEDs) affixed to the screw guide to indicate a relative direction from a current angle of the screw guide to the specified angle during tracking.
Example 25 is a system for aligning a screw guide for inserting a spinal screw at a specified angle comprising: an articulated arm including a locking mechanism; a first sensor, affixed to a distal end of the articulated arm; a screw guide, affixed to the distal end of the articulated arm; processing circuitry configured to determine that the screw guide is configured at the specified angle and cause the locking mechanism to lock the articulated arm with the screw guide at the specified angle.
In Example 26, the subject matter of Example 25 includes, wherein the first sensor is an inertial measurement unit.
In Example 27, the subject matter of Examples 25-26 includes, a second sensor that is housed within a same housing as a marker, the marker configured to define a coordinate system with respect to the patient using a diagnostic image of the patient.
In Example 28, the subject matter of Examples 25-27 includes, wherein the processing circuitry is housed in the articulated arm.
In Example 29, the subject matter of Examples 25-28 includes, wherein the processing circuitry is remote from the articulated arm.
In Example 30, the subject matter of Examples 25-29 includes, wherein the screw guide includes a depth sensor, the depth sensor configured to output information indicating a depth of a tool inserted into the screw guide.
In Example 31, the subject matter of Examples 25-30 includes, a graphical user interface that indicates a relative angle of the screw guide with respect to the specified position and angle during tracking.
In Example 32, the subject matter of Examples 25-31 includes, wherein a set of light emitting diodes (LEDs) indicate a relative position and orientation of the screw guide with respect to the specified position and orientation.
Example 33 is a method for aligning a guide for inserting a screw at a specified angle comprising: tracking, using processing circuitry, movement of the guide affixed to a distal end of an articulated arm with respect to a reference point based on sensor data generated by a first sensor affixed to the distal end of the articulated arm; determining, using the processing circuitry, whether the articulated arm is at an orientation such that the guide is arranged at the specified angle, based on the sensor data; and causing, in response to determining that the guide is arranged at the specified angle, a locking mechanism of the articulated arm to lock the articulated arm at the orientation.
Example 34 is a method for aligning a guide for inserting a screw at a specified angle comprising: receiving information indicative of a reference point from a first sensor secured to a patient; tracking, using processing circuitry, movement of the guide affixed to a distal end of an articulated arm with respect to the reference point based on sensor data generated by a second sensor affixed to the distal end of the articulated arm and the information indicative of the reference point; and determining, using the processing circuitry, whether the articulated arm is at an orientation such that the guide is arranged at the specified angle, based on the sensor data and the information indicative of the reference point.
In Example 35, the subject matter of Example 34 includes, registering the reference point to a preoperative patient image based on a radio-opaque marker within a housing of the first sensor.
In Example 36, the subject matter of Example 35 includes, wherein the specified angle is a screw entry angle preplanned using the preoperative patient image.
In Example 37, the subject matter of Examples 1-36 includes, wherein tracking the movement includes receiving accelerometer data, gyroscope data, and magnetometer data from the second sensor, the second sensor being an inertial measurement unit (IMU).
Example 38 is at least one machine-readable medium including instructions that, when executed by processing circuitry, cause the processing circuitry to perform operations to implement of any of Examples 1-37.
Example 39 is an apparatus comprising means to implement of any of Examples 1-37.
Example 40 is a system to implement of any of Examples 1-37.
Example 41 is a method to implement of any of Examples 1-37.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A system for aligning a screw guide for inserting a spinal screw at a specified angle comprising:
processing circuitry and memory, including instructions, which when executed by the processing circuitry, cause the processing circuitry to:
track movement of the screw guide affixed to a distal end of an articulated arm with respect to a reference point based on sensor data generated by a first sensor affixed to the distal end of the articulated arm;
determine whether the articulated arm is at an orientation such that the screw guide is arranged at the specified angle, based on the sensor data; and
cause, in response to determining that the screw guide is arranged at the specified angle, a locking mechanism of the articulated arm to lock the articulated arm at the orientation.

2. The system of claim 1, wherein to track the movement of the screw guide, the processing circuitry is further configured to receive information indicative of the reference point from a second sensor secured to a patient.

3. The system of claim 2, wherein the instructions further cause the processing circuitry to register the reference point to a preoperative patient image based on a radio-opaque marker within a housing of the second sensor.

4. The system of claim 3, wherein the specified angle is a screw entry angle preplanned using the preoperative patient image.

5. The system of claim 1, wherein to track the movement of the screw guide, the processing circuitry is further configured to receive accelerometer data, gyroscope data, and magnetometer data from the first sensor, the first sensor being an inertial measurement unit (IMU).

6. The system of claim 1, wherein the instructions further cause the processing circuitry to receive depth information, from a third sensor affixed to the screw guide, for a tool inserted into the screw guide, the depth information corresponding to a screw bore depth.

7. The system of claim 1, wherein the instructions further cause the processing circuitry to output for display, on a graphical user interface (GUI), an current angle of the screw guide and the specified angle of the screw guide during tracking.

8. The system of any of claims 1-8, wherein the instructions further cause the processing circuitry to activate one or more of a plurality of light emitting diodes (LEDs) affixed to the screw guide to indicate a relative direction from a current angle of the screw guide to the specified angle during tracking.

9. A method for aligning a screw guide for inserting a spinal screw at a specified angle comprising:
tracking, using processing circuitry, movement of the screw guide affixed to a distal end of an articulated arm with respect to a reference point based on sensor data generated by a first sensor affixed to the distal end of the articulated arm;
determining, using the processing circuitry, whether the articulated arm is at an orientation such that the screw guide is arranged at the specified angle, based on the sensor data; and
causing, in response to determining that the screw guide is arranged at the specified angle, a locking mechanism of the articulated arm to lock the articulated arm at the orientation.

10. The method of claim 9, wherein tracking the movement of the screw guide includes receiving information indicative of the reference point from a second sensor secured to a patient.

11. The method of claim 10, further comprising registering the reference point to a preoperative patient image based on a radio-opaque marker within a housing of the second sensor.

12. The method of claim 9, further comprising receiving depth information, from a third sensor affixed to the screw guide, for a tool inserted into the screw guide, the depth information corresponding to a screw bore depth.

13. The method of claim 9, further comprising displaying, on a graphical user interface (GUI), a current angle of the screw guide and the specified angle of the screw guide during tracking.

14. The method of any of claims 9-13, further comprising activating one or more of a plurality of light emitting diodes (LEDs) affixed to the screw guide to indicate a relative direction from a current angle of the screw guide to the specified angle during tracking.

15. At least one machine-readable medium including instructions for operation of a computing system, which when executed by a machine, cause the machine to perform operations of any of the methods of claims 9-13.
